# EUROPÄISCHE PATENTANMELDUNG

(11) **EP 0 923 948 A2**
(43) Veröffentlichungstag der Anmeldung: **23.06.1999**
(21) Anmeldenummer: 98123403.2
(22) Anmeldetag: 09.12.1998
(51) Int. Cl.: A61L 2/18, G02C 13/00

(54) **Vorrichtung zur Pflege von Kontaktlinsen**

(30) Priorität: 22.12.1997 DE 19757356
(71) Anmelder: MDLE Medical Device Laboratories Europe GmbH, 87700 Memmingen (DE)
(72) Erfinder: Müller-Lierheim, Wolfgang G.K. Dr., 81477 München (DE)
(74) Vertreter: Nöth, Heinz, Dipl.-Phys.

(57) **Zusammenfassung**

Eine Vorrichtung zur Pflege von Kontaktlinsen mit wenigstens einem napfförmigen Behälter 1 aus Glas, dessen Innenseite eine Platinschicht 2 trägt, wobei der Behälter 1 auswechselbar in ein Gehäuse 4 einsetzbar ist und in den Behälter 1 eine H₂O₂-Pflegelösung einfüllbar ist. Ein Deckel 3 wird mit dem Gehäuse 4 beim Verschließen gasdicht verbunden.

## Beschreibung

Die Erfindung betrifft eine Vorrichtung zur Pflege von Kontaktlinsen mit wenigstens einem Behälter aus Glas, auf dessen Innenseite eine Platinschicht aufgebracht ist, und mit einer in den Behälter einfüllbaren wässrigen H₂O₂-Pflegelösung zur Sterilisierung und/oder Desinfektion und einem Deckel zum Verschließen des Behälters.

Eine derartige Vorrichtung ist aus der DE 196 24 095 C1 bekannt. Wie ferner aus der deutschen Patentschrift DE 24 25 714 B2 bekannt ist, können Kontaktlinsen, insbesondere Weichkontaktlinsen, mittels Wasserstoffperoxyd in einer wässrigen insbesondere 3%-wässrigen Wasserstoffperoxydlösung sterilisiert werden. Zur Beseitigung von Resten des Wasserstoffperoxyds werden diese mit Hilfe eines Zersetzungskatalysators in Wasser und Sauerstoff zersetzt. Als Katalysator wirkt bei dieser Zersetzung die Platinschicht in dem Behälter, welcher aus der DE 196 24 095 C1 bekannt ist. Bei der Kontaktlinsenpflege und bei der katalytischen Zersetzung des Wasserstoffperoxyds entstehen Gase.

Aus der US 4,889,693 ist eine eingangs genannte Vorrichtung zur Kontaktlinsenpflege bekannt, bei welcher in dem auf den Behälter aufschraubbaren Deckel Öffnungen zum Entweichen des entstehenden Gases vorgesehen sind. Bei einer aus der DE 32 30 231 A1 bekannten Vorrichtung der eingangsgenannten Art sind im Behälter Öffnungen vorgesehen, durch die das Gas entweichen kann. Im Bereich der Öffnungen ist eine Abdeckung vorgesehen, welche dampfdurchlässig ist, jedoch eine flüssigkeitsundurchlässige Sperre bildet. Aus der GB 2 209 845 ist es bekannt, im Behälter ein Überdruckventil zum Entweichen der entstehenden Gase vorzusehen. Bei der aus der US 4,996,027 bekannten Vorrichtung ist zwischen dem oberen Rand des Behälters und dem aufgeschraubten Deckel eine Dichtung vorsehen, welche bei entstehendem Gasdruck verformt wird, so daß überschüssiges Gas entweichen kann. Bei den bekannten Vorrichtungen sind die Deckel auf die Behälter aufgeschraubt, in denen die wässrige H₂O₂ - Plegelösung eingefüllt ist. Beim Gasaustritt mitgerissene Flüssigkeitsteilchen treten nach außen hin in Erscheinung.

Aufgabe der Erfindung ist es, eine Vorrichtung der eingangs genannten Art zu schaffen, welche eine einfache Handhabung bietet und bei welcher die während der Kontaktlinsenpflege und der Neutralisation der Pflegelösung entstehenden Gase ohne Beeinträchtigung des äußeren Erscheinungsbildes der Vorrichtung entweichen können.

Diese Aufgabe wird erfindungsgemäß durch die im Patentanspruch 1 angegebenen Merkmale gelöst. Hierzu besitzt die Vorrichtung ein Gehäuse, in welches der Behälter auswechselbar in ein Aufnahmefach einsetzbar ist. Der Deckel ist beim Verschließen mit dem Gehäuse gasdicht verbunden und zwischen dem oberen Rand des Behälters und der Deckelinnenseite sowie zwischen der Außenseite des Behälters und der Innenseite des Aufnahmefaches ein Zwischenraum für einen Gasdurchlaß vorgesehen ist.

Das Gehäuse ist so ausgebildet, daß das Gas nach außen entweichen kann. Beispielsweise besitzt das Gehäuse an seiner Unterseite einen aufklipsbaren, aufschiebbaren oder sonstwie befestigbaren Boden, wobei zwischen Gehäuse und Boden ein Spalt verbleibt, durch welchen das Gas entweichen kann. In bevorzugter Weise besteht das Gehäuse aus Kunststoff. Auch der Deckel, welcher auf das Gehäuse aufschraubbar sein kann, kann aus Kunststoff bestehen. Durch die Schraubverbindung zwischen dem Gehäuse und dem Deckel wird über dem Glasbehälter, in welchem die Kontaktlinsenpflege in der H₂O₂-Pflegelösung stattfindet, ein gasdichter Verschluß erzielt. Die Schraubgewinde für die Schraubverbindung zwischen dem Deckel und dem Gehäuse sind an der Innenseite des Deckels an einem umlaufenden Rand und an einem die Öffnung des Aufnahmefaches umgebenden am Gehäuse hochstehenden Rand vorgesehen. In bevorzugter Weise besitzen der vorzugsweise napfförmige Behälter und das Aufnahmefach einen kreisrunden Querschnitt, wobei der Innendurchmesser des Aufnahmefaches größer bemessen ist als der Außendurchmesser des Behälters. Der obere Rand des in das Aufnahmefach eingesetzten Behälters kann etwas tiefer liegen als der obere das Aufnahmefach umgebende Rand am Gehäuse, welcher das Gewinde aufweist. Hierdurch wird, wie bei einem Überlauf, ein Entweichen des Gases aus dem Glasbehälter ermöglicht. Am Deckel und am Gehäuse müssen keine gesonderten Entgasungsöffnungen oder Ventile vorgesehen sein.

Die Platinschicht ist auf der Innenseite des Glasbehälters vorzugweise durch Sputtern aufgebracht. Die Platinschicht kann in der Weise ausgebildet sein, wie es in der DE 196 24 095 C1 beschrieben ist. In bevorzugter Weise besitzt die Platinschicht eine solche Ausgestaltung, daß sie über die Neutralisation des Wasserstoffperoxyds hinaus eine langanhaltende Konservierungswirkung entfaltet. Neben der erwünschten hohen Desinfektionswirkung des H₂O₂-Pflegemittels in Form der wässrigen Lösung erreicht man nach der Neutralisation der Lösung, daß ein Wachstum von Mikroorganismen vermieden wird. Dies beruht darauf, daß aus der dünnen aufgesputterten Platinschicht eine solche Platinmenge herausgelöst wird, daß die Lösung die Wirkung einer Konservierungslösung hat, so daß die Linse langzeitlich in dieser Lösung aufbewahrt werden kann. In vorteilhafter Weise erreicht man einen antimikrobiellen sogenannten oligodynamischen Effekt in der neutralisierten Pflegelösung. Die Zugabe eines Konservierungsmittels ist daher nicht erforderlich.

Der jeweilige insbesondere napfförmige Behälter mit der Platinschicht kann bei der Verwendung einer jeweils neuen Vorratsflasche mit H₂O₂-Pflegelösung ausgewechselt werden. Jeder Vorratsflasche kann ein napfförmiger Behälter, in welchem zwei Kontaktlinsen gepflegt werden können, beigefügt sein. Es können auch zwei Behälter je für eine Kontaktlinse zur Bildung eines Pflegemittelsatzes beigefügt sein. Es ist natürlich auch möglich, den bzw. die beiden Behälter mit dem Gehäuse zusammen mit der Vorratsflasche als Pflegemittelsatz in Bereitschaff zu halten.

Anhand der Figuren wird die Erfindung noch näher erläutert. Es zeigt:
- Fig. 1: ein erstes Ausführungsbeispiel, und
- Fig. 2: ein zweites Ausführungsbeispiel der Erfindung.

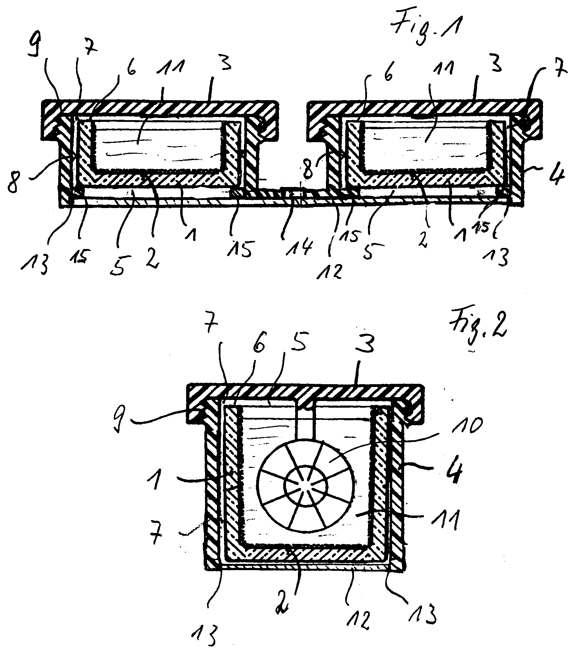

Die beiden Ausführungsbeispiele der Fig. 1 und 2 sind schematische schnittbildliche Darstellungen. Beim ersten Ausführungsbeispiel der Fig. 1 sind in einem Gehäuse 4 in zwei Aufnahmefächern 5 des Gehäuses zwei napfförmige Behälter 1 aus Glas, welche flüssigkeitsundurchlässig sind, angeordnet. Beim Ausführungsbeispiel der Fig. 2 befindet sich ein Glasbehälter 1 im Gehäuse 4 in dem Aufnahmefach 5. An der Außenseite jedes Aufnahmefaches 5 ist am oberen Rand ein Schraubgewinde vorgesehen. Ferner ist an der Innenseite eines jeden Deckels ein Schraubgewinde vorgesehen. Hierdurch wird eine gasdichte Verbindung zwischen dem Deckel und dem Gehäuse 4 mittels Schraubverbindung 9 hergestellt. Innerhalb der Aufnahmefächer 5 werden die jeweiligen Behälter 1 im Abstand von der Deckelunterseite und von der Innenfläche des jeweiligen Aufnahmefaches 5 gehalten. Hierdurch entsteht zwischen dem jeweiligen oberen Rand 6 des Behälters 1 und zwischen der jeweiligen Behälteraußenseite und der Innenseite des jeweiligen Aufnahmefaches 5 ein Zwischenraum bzw. Zwischenräume 7, die einen Durchlaß für während der Kontaktlinsenpflege in den jeweiligen Behältern 1 bilden.

Am jeweiligen Gehäuse 4 ist an der Unterseite ein Boden 12 vorgesehen. Während das Gehäuse 4 aus Kunststoff bestehen kann, besteht der Boden 12 in bevorzugter Weise aus Metall oder metallisiertem Kunststoff. Der Boden kann dann beim Einsetzen und Herausnehmen der Kontaktlinsen in die Augen bzw. aus den Augen als Spiegel zu Hilfe genommen werden. Der Boden 12 kann mit Hilfe einer Schnappverbindung 14 mit dem Gehäuse 4 verbunden sein. Zwischen einem umlaufenden Rand des Bodens 12 und dem Gehäuse 4 kann durch die Schnappverbindung oder einer ähnlichen Verbindung ein Gasdurchtrittspalt 13 vorhanden sein. Hierdurch ist gewährleistet, daß Gas, welches während der Kontaktlinsenpflege bzw. während der Aufbewahrung der Kontaktlinse in einer wässrigen Pflegelösung 11 im Behälter 10 entstanden und durch die Zwischenräume 7 aus den jeweiligen Behältern 1 entwichen ist, nach außen durch die Gasdurchtrittsspalte 13 gelangen kann.

Mit Hilfe von Abstandhaltern 8 und Auflagevorsprüngen 15, die in Winkelabständen voneinander an der Innenfläche des Aufnahmefaches 5 vorgesehen sind, werden die Behälter 1 aus Glas in den jeweiligen Aufnahmefächern 5 des Gehäuses 4 gehalten.

Die Glasbehälter 1 können auch mit einem abgerundeten Boden wie es beispielsweise aus der EP 0 049 767 A2 bei dicht bzw. fest in das Gehäuse eingesetzten Kunststoffbehältern bekannt ist, ausgebildet sein.

Der Glasbehälter 1 ist an seiner Innenseite ganz oder teilweise mit einer Platinschicht 2 versehen. Diese Platinschicht 2 kann so ausgestattet sein, wie es aus der deutschen Patentschrift DE 196 24 095 bekannt ist. Die Platinschicht wirkt als Zersetzungskatalysator für die wässrige Pflegelösung 11, welche bei den dargestellten Ausführungsbeispielen eine wässrige H₂O₂-Pflegelösung ist. In bekannter Weise kann die Wasserstoffperoxydlösung als 3%ige H₂O₂-Lösung ausgebildet sein. Durch die Wirkung dieser Pflegelösung werden die eingetauchten Kontaktlinsen sterilisiert und desinfiziert. Um Wasserstoffperoxydreste an den Kontaktlinsen nach der Pflege zur vermeiden, wird durch die Wirkung der Platinschicht 2 das Wasserstoffperoxyd in Wasserstoff und Sauerstoff zersetzt (neutralisiert). Nach dieser Neutralisierung wirkt die Platinschicht, welche insbesondere durch Sputtern auf die gegebenenfalls aufgerauhte Oberfläche des Glasbehälters 1 aufgebracht ist, antimikrobiell (oligodynamisch), so daß die neutralisierte Lösung in Zusammenwirkung mit der Platinschicht ein Konservierungssystem für die aufbewahrten Linsen bildet.

Die jeweiligen Glasbehälter 1 mit den aufgesputterten Platinschichten 2 können auch als Pflegemittelsatz gegebenenfalls mit den Behältern 4 zusammen mit einem Vorratsgefäß, in welchem sich die wässrige insbesondere 3%-ige H₂O₂-Pflegelösung befindet, in Bereitschaft gehalten werden.

Während beim Ausführungsbeispiel der Fig. 1 zwei Glasbehälter 1 für die Pflege der beiden Kontaktlinsen vorgesehen sind, werden beim Ausführungsbeispiel der Fig. 2 die beiden Kontaktlinsen in einem Linsenhalter 10, welcher an der Unterseite des Deckels 3 befestigt sein kann, in die wässrige Pflegelösung 11 im Glasbehälter 2 eingetaucht.

Zum Eintauchen der Linsen in die H₂O₂-Pflegelösung kann der Linsenhalter 10 in Form von Körbchen, welche die zu pflegenden Linsen aufnehmen, ausgebildet sein. Als Linsenhalter 10 können jedoch auch mehrere Vorsprünge, beispielsweise in Form von Zapfen, an der Unterseite des Deckels vorgesehen sein, durch welche ein Eintauchen der Linsen in die Pflegelösung 11 gewährleistet wird.

Bei dem in der Fig. 1 dargestellten Ausführungsbeispiel können an die Unterseite des jeweiligen Deckels 3 Vorsprünge oder ein sternförmig ausgebildetes kalottenförmig nach unten ragendes Profil vorgesehen sein, wie es aus der EP 0 049 767 bekannt ist.

## Patentansprüche

1. Vorrichtung zur Pflege von Kontaktlinsen, mit
- wenigstens einem Behälter (1) aus Glas, auf dessen Innenseite eine Platinschicht aufgebracht ist,
- einer in den Behälter (1) einfüllbaren wässrigen H₂O₂-Pflegelösung zur Kontaktlinsenpflege durch Sterilisierung und/oder Desinfektion,
- einem Deckel (3) zum Verschließen des Behälters (1), und
- einem Gehäuse (4) mit einem Aufnahmefach (5) in das der Behälter (1) auswechselbar einsetzbar ist,
wobei der Deckel (3) beim Verschließen mit dem Gehäuse (4) gasdicht verbunden ist und zwischen dem oberen Rand (6) des Behälters (1) und dem Deckel (3) sowie zwischen der Außenseite des Behälters (1) und der Innenseite des Aufnahmefaches (5) ein Zwischenraum (7) für einen Gasdurchlaß vorgesehen ist.

2. Vorrichtung nach Anspruch 1, dadurch gekennzeichnet, daß der Deckel (3) einen abnehmbaren Boden aufweist, wobei im Bereich des Umfangrandes des Bodens zwischen dem Boden und dem Gehäuse ein Gasdurchtrittsspalt (13) gebildet ist.

3. Vorrichtung nach Anspruch 1 oder 2, dadurch gekennzeichnet, daß der Deckel (3) auf das Gehäuse (4) aufschraubbar ist, wobei eine Schraubverbindung (9) zwischen dem Deckel (3) und dem Gehäuse (4) das Aufnahmefach (5), in welchem der Glasbehälter (1) angeordnet ist, umfaßt.

4. Vorrichtung nach einem der Ansprüche 1 bis 3, dadurch gekennzeichnet, daß der Deckel und/oder das Gehäuse aus Kunststoff bestehen.

5. Vorrichtung nach einem der Ansprüche 1 bis 4, dadurch gekennzeichnet, daß der Boden (12) aus Metall besteht.

6. Vorrichtung nach einem der Ansprüche 1 bis 5, dadurch gekennzeichnet, daß im Gehäuse (4) ein oder zwei auswechselbare Glasbehälter (1) vorgesehen sind.

7. Vorrichtung nach einem der Ansprüche 1 bis 6, dadurch gekennzeichnet, daß die Platinschicht (2) in der Weise durch Sputtern auf die Innenfläche des Behälters (1) aufgebracht ist, daß eine konservierend wirkende Menge, welche ein mikrobielles Wachsen hemmt, in die durch die Platinschicht katalytisch neutralisierte H₂O₂-Pflegelösung abgegeben ist.

8. Vorrichtung nach einem der Ansprüche 1 bis 7, dadurch gekennzeichnet, daß durch ein Vorratsgefäß für die H₂O₂-Pflegelösung und einem oder zwei in das Gehäuse (4) einsetzbaren Glasbehältern (1) ein Pflegemittelsatz gebildet ist.

9. Vorrichtung nach einem der Ansprüche 1 bis 8, dadurch gekennzeichnet, daß Mittel (10) zum Eintauchen der Kontaktlinsen in die H₂O₂-Pflegelösung an der Deckelunterseite vorgesehen sind.
